# EUROPEAN PATENT APPLICATION

(11) **EP 3 851 053 A1**
(43) Date of publication of application: **21.07.2021**
(21) Application number: 21151534.1
(22) Date of filing: 14.01.2021
(51) Int. Cl.: A61B 8/08, A61B 8/12, A61B 8/00

(54) **ULTRASOUND IMAGING APPARATUS**

(30) Priority: 17.01.2020 KR 20200006550
(71) Applicant: Samsung Medison Co., Ltd., Hongcheon-gun, Gangwon-do 25108 (KR)
(72) Inventor: LEE, Dongeun, 05340 Seoul (KR); LEE, Jin-Yong, 05340 Seoul (KR)
(74) Representative: Arnold & Siedsma

(57) **Abstract**

Provided are an ultrasound imaging apparatus for determining a Bishop score through ultrasound and displaying the Bishop score, and a method of controlling the same. The ultrasound imaging apparatus includes: a display; a probe configured to transmit an ultrasound signal to an object and detect an echo signal; and a controller configured to generate an ultrasound image of a cervix of the object and a fetus on the basis of the echo signal, acquire state information of the cervix and position information of the fetus by processing the ultrasound image, determine a numeric value indicating a degree of delivery progression on the basis of the state information of the cervix and the position information of the fetus, and control the display to display the numeral value.

## Description

### CROSS-REFERENCE TO RELATED APPLICATION

This application is based on and claims priority under 35 U.S.C. §119 to Korean Patent Applications No. 10-2020-0006550, filed on January 17, 2020 in the Korean Intellectual Property Office, the disclosures of which are incorporated herein by reference.

### BACKGROUND

### 1. Field

The disclosure relates to an ultrasound imaging apparatus for providing an ultrasound image by irradiating an ultrasound signal onto an object and receiving an echo signal reflected from the object, and a control method thereof.

### 2. Description of the Related Art

In general, in medical fields, a Bishop score calculated based on changes in the cervix and the positi0on of the fetus is used as a measure of the degree of delivery progress.

In order to calculate the Bishop score, a medical practitioner may conduct an endoscopy examination on the mother, which may cause pain or discomfort in the mother.

In addition, since the bishop score through endoscopy is calculated on the basis of subjective judgment by a medical practitioner, the calculation of the bishop score may be inaccurate and have a low reliability.

### SUMMARY

Therefore, it is an object of the disclosure to provide an ultrasound imaging apparatus capable of determining a Bishop score through ultrasound and displaying the Bishop score, and a method of controlling the same.

Additional aspects of the disclosure will be set forth in part in the description which follows and, in part, will be obvious from the description, or may be learned by practice of the disclosure.

According to an aspect of the disclosure, there is provided an ultrasound imaging apparatus including: a display; a probe configured to transmit an ultrasound signal to an object and detect an echo signal; and a controller configured to generate an ultrasound image of a cervix of the object and a fetus on the basis of the echo signal, acquire state information of the cervix and position information of the fetus by processing the ultrasound image, determine a numeric value indicating a degree of delivery progression on the basis of the state information of the cervix and the position information of the fetus, and control the display to display the numeral value.

The state information of the cervix may include a length of the cervix, a degree to which the cervix is dilated, a stiffness of the cervix, and a position of the cervix.

The controller may determine the numeral value indicating the degree of delivery progression by summating numeral values corresponding respectively to the length of the cervix, the degree to which the cervix is dilated, the stiffness of the cervix, the position of the cervix, and the position information of the fetus.

The controller, in response to information regarding each of the length of the cervix, the stiffness of the cervix, and the position of the cervix being acquired by processing the ultrasound image, may determine the numeric value corresponding to the degree to which the cervix is dilated to a minimum value.

The controller, in response to the degree to which the cervix is dilated being acquired by processing the ultrasound image, may determine the numeric values corresponding respectively to the length of the cervix, the stiffness of the cervix, and the position of the cervix to maximum values.

The probe may include: a first probe configured to be inserted into a body of the object to detect the echo signal; and a second probe configured to come in contact with a surface of the object to detect the echo signal.

The controller may acquire the position of the cervix, the stiffness of the cervix, and the length of the cervix by processing the ultrasound image generated on the basis of the echo signal of the first probe.

The controller may acquire the degree to which the cervix is dilated and the position information of the fetus by processing the ultrasound image generated on the basis of the echo signal of the second probe.

The controller may control the display to display at least one of the state information of the cervix or the position information of the fetus.

The ultrasound imaging apparatus may further include an inputter configured to receive a user input, wherein the controller may adjust at least one of the state information of the cervix or the position information of the fetus on the basis of the user input received through the inputter.

According to another aspect of the disclosure, there is provided a method of controlling an ultrasound imaging apparatus including a display and a probe configured to transmit an ultrasound signal to an object and detect an echo signal, the method including: generating an ultrasound image of a cervix of the object and a fetus on the basis of the echo signal; acquiring state information of the cervix and position information of the fetus by processing the ultrasound image; determining a numeric value indicating a degree of delivery progression on the basis of the state information of the cervix and the position information of the fetus; and controlling the display to display the numeral value.

The state information of the cervix may include a length of the cervix, a degree to which the cervix is dilated, a stiffness of the cervix, and a position of the cervix.

The determining of the numeric value indicating the degree of delivery progression on the basis of the state information of the cervix and the position information of the fetus may include determining the numeral value indicating the degree of delivery progression by summating numeral values corresponding respectively to the length of the cervix, the degree to which the cervix is dilated, the stiffness of the cervix, the position of the cervix, and the position information of the fetus.

The determining of the numeric value indicating the degree of delivery progression on the basis of the state information of the cervix and the position information of the fetus may include, in response to information regarding each of the length of the cervix, the stiffness of the cervix, and the position of the cervix being acquired by processing the ultrasound image, determining the numeric value corresponding to the degree to which the cervix is dilated to a minimum value.

The determining of the numeric value indicating the degree of delivery progression on the basis of the state information of the cervix and the position information of the fetus may include, in response to the degree to which the cervix is dilated being acquired by processing the ultrasound image, determining the numeric values corresponding respectively to the length of the cervix, the stiffness of the cervix, and the position of the cervix to maximum values.

The probe may include a first probe configured to be inserted into a body of the object to detect the echo signal; and a second probe configured to come in contact with a surface of the object to detect the echo signal.

The acquiring of the state information of the cervix and the position information of the fetus by processing the ultrasound image may include acquiring the position of the cervix, the stiffness of the cervix, and the length of the cervix by processing the ultrasound image generated on the basis of the echo signal of the first probe.

The acquiring of the state information of the cervix and the position information of the fetus by processing the ultrasound image may include acquiring the degree to which the cervix is dilated and the position information of the fetus by processing the ultrasound image generated on the basis of the echo signal of the second probe.

The method may further include controlling the display to display at least one of the state information of the cervix or the position information of the fetus.

The ultrasound imaging apparatus may further include an inputter configured to receive a user input, wherein the method may further include adjusting at least one of the state information of the cervix or the position information of the fetus on the basis of the user input received through the inputter.

### BRIEF DESCRIPTION OF THE DRAWINGS

These and/or other aspects of the disclosure will become apparent and more readily appreciated from the following description of the embodiments, taken in conjunction with the accompanying drawings of which:
FIG. 1 is a diagram illustrating an external appearance of an ultrasound imaging apparatus according to an embodiment of the disclosure;
FIG. 2 is a control block diagram illustrating the ultrasound imaging apparatus according to the embodiment of the disclosure;
FIG. 3 is a diagram for describing a case in which the ultrasound imaging apparatus according to the embodiment determines a numerical value indicating the degree of delivery progress;
FIG. 4A is diagram illustrating a case in which the ultrasound imaging apparatus according to the embodiment determines the position of a cervix;
FIG. 4B is diagram illustrating a case in which the ultrasound imaging apparatus according to the embodiment determines the position of a cervix;
FIG. 5 is a diagram illustrating numerical values for the positions of the cervix;
FIG. 6 is a diagram illustrating a case in which the ultrasound imaging apparatus according to the embodiment of the disclosure determines stiffness of the cervix;
FIG. 7A is a diagram for describing the length of the cervix and the degree of the cervix dilation;
FIG. 7B is a diagram for describing the length of the cervix and the degree of the cervix dilation;
FIG. 7C is a diagram for describing the length of the cervix and the degree of the cervix dilation;
FIG. 7D is a diagram for describing the length of the cervix and the degree of the cervix dilation;
FIG. 8 is a diagram illustrating a case in which the ultrasound imaging apparatus according to the embodiment of the disclosure determines the length of the cervix;
FIG. 9 is a diagram illustrating a case in which the ultrasound imaging apparatus according to the embodiment determines the degree to which the cervix is dilated;
FIG.10A is a diagram illustrating a case in which the ultrasound imaging apparatus according to the embodiment of the disclosure determines position information of a fetus;
FIG. 10B is a diagram illustrating a case in which the ultrasound imaging apparatus according to the embodiment of the disclosure determines position information of a fetus;
FIG. 11 is a diagram illustrating a case in which the ultrasound imaging apparatus according to the embodiment of the disclosure displays a numerical value indicating the degree of delivery progress;
FIG. 12 is a flowchart showing a method of controlling an ultrasound imaging apparatus according to an embodiment of the disclosure, which shows a case of displaying a numerical value indicating the degree of delivery progress; and
FIG. 13 is a flowchart showing a method of controlling an ultrasound imaging apparatus according to an embodiment of the disclosure, which shows a case of determining a numerical value indicating the degree of delivery progression on the basis of a user input.

### DETAILED DESCRIPTION

The embodiments set forth herein and illustrated in the configuration of the present disclosure are only the most preferred embodiments and are not representative of the full the technical spirit of the present disclosure, so it should be understood that they may be replaced with various equivalents and modifications at the time of the disclosure.

It will be further understood that the term "connect" or its derivatives refer both to direct and indirect connection, and the indirect connection includes a connection over a wireless communication network.

The terms used herein are for the purpose of describing the embodiments and are not intended to restrict and/or to limit the present disclosure. For example, the singular expressions herein may include plural expressions, unless the context clearly dictates otherwise. Also, the terms "comprises" and "has" are intended to indicate that there are features, numbers, steps, operations, elements, parts, or combinations thereof described in the specification, and do not exclude the presence or addition of one or more other features, numbers, steps, operations, elements, parts, or combinations thereof.

It will be understood that, although the terms first, second, etc. may be used herein to describe various components, these components should not be limited by these terms. These terms are only used to distinguish one component from another. For example, without departing from the scope of the present disclosure, the first component may be referred to as a second component, and similarly, the second component may also be referred to as a first component. The term "and/or" includes any combination of a plurality of related items or any one of a plurality of related items.

Moreover, terms described in the specification such as "part," "module," and "unit," refer to a unit of processing at least one function or operation, and may be implemented by software, a hardware component such as a field-programmable gate array (FPGA) or an application-specific integrated circuit (ASIC), or a combination of software and hardware.

Reference numerals used for method steps are just used for convenience of explanation, but not to limit an order of the steps. Thus, unless the context clearly dictates otherwise, the written order may be practiced otherwise

Hereinafter, embodiments according to the disclosure will be described in detail with reference to the accompanying drawings.

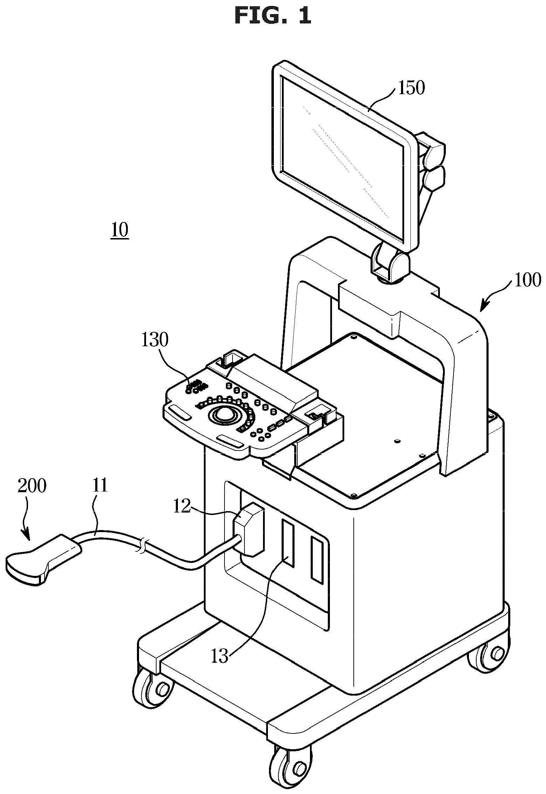

FIG. 1 is a diagram illustrating an external appearance of an ultrasound imaging apparatus according to an embodiment of the disclosure.

Referring to FIG. 1, an ultrasound imaging apparatus 10 according to an embodiment may include an ultrasound probe 200 that transmits an ultrasound signal to an object, receives an ultrasound echo signal from the object, and converts the echo signal into an electrical signal, and a main body 100 including an inputter 130 connected to the ultrasound probe 200 and receiving an input from a user and a display 150 configured to display an ultrasound image.

The ultrasound probe 200 according to the embodiment may be connected to the main body 100 through a cable 11 to receive various signals required for controlling the ultrasound probe 200, or transmit an analog signal or digital signal corresponding to the ultrasound echo received by the ultrasound probe 200 to the main body 100.

To this end, the cable 11 has one side end connected to the ultrasound probe 200, and the other side end connected to a connector 12 couplable to or separable from a slot 13 of the main body 100. That is, the main body 100 and the ultrasound probe 200 may exchange control commands or data using the cable 11.

However, the embodiment of the ultrasound probe 200 is not limited thereto, and the ultrasound probe 200 may be implemented as a wireless probe to transmit and receive signals through a wireless network formed between the ultrasound probe 200 and the main body 100.

That is, when the ultrasound probe 200 is implemented as a wireless probe, the ultrasound probe 200 may be connected to the main body 100 through a wireless network instead of the cable 11, and the main body 100 and the ultrasound probe 200 may exchange control commands or data via a wireless network.

In this case, the ultrasound probe 200 may be any one of an endocavity transducer, a convex array transducer, or a linear array transducer.

In addition, although one ultrasound probe 200 is connected to the main body 100 in FIG. 1, the disclosure is not limited thereto, and a plurality of the ultrasound probes 200 may be connected to one main body 100.

For example, a first probe configured to be inserted into the body of the object to detect an echo signal and a second probe configured to come in contact with the surface of the body of the object to detect an echo signal may be connected to the main body 100. That is, according to the embodiment, the ultrasound probe 200 may include the first probe that is inserted into the body of an object to detect an ultrasound echo signal, and the second probe that comes in contact with the body surface of the object to detect an echo signal. In this case, the first probe may be an endo cavity transducer, and the second probe may be a convex array transducer or a linear array transducer.

Meanwhile, a plurality of casters for mobility of the ultrasound imaging apparatus 10 may be provided at a lower side of the main body 100. The plurality of casters may allow the ultrasound imaging apparatus 10 to be fixed at a specific place or be moved in a specific direction. Such an ultrasound imaging apparatus 10 is referred to as a cart type ultrasound imaging apparatus.

However, unlike FIG. 1, the ultrasound imaging apparatus 10 according to the embodiment may be a portable ultrasound imaging apparatus that may be carried for a long-distance movement. In this case, the portable ultrasound imaging apparatus may not include a caster. Examples of the portable ultrasound imaging apparatus may include a picture archiving and communication system (PACS) viewer, a smart phone, a laptop computer, a personal digital assistant (PDA), a tablet personal computer (a tablet PC), and the like, but are not limited thereto.

Hereinafter, components of the main body 100 and the ultrasound probe 200 of the ultrasound imaging apparatus 10 will be described in detail.

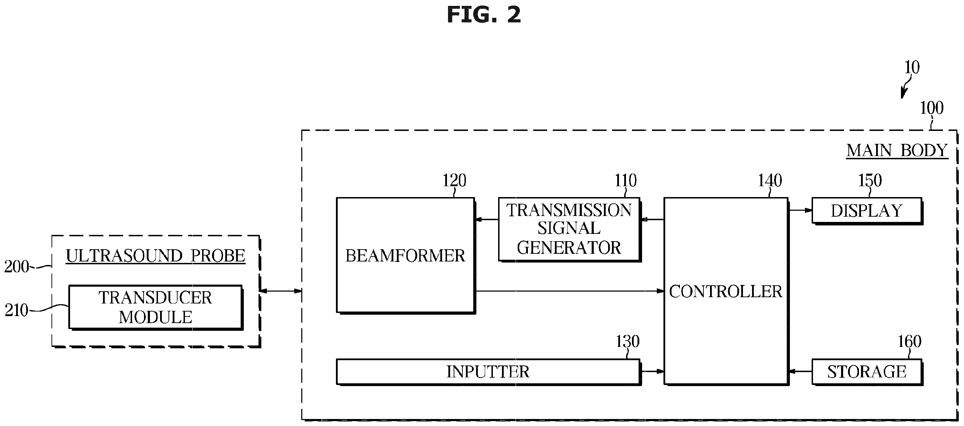

FIG. 2 is a control block diagram illustrating the ultrasound imaging apparatus 10 according to the embodiment of the disclosure.

Referring to FIG. 2, the ultrasound probe 200 of the ultrasound imaging apparatus 10 according to the embodiment includes a transducer module 210 including an ultrasound transducer array.

The transducer module 210 according to the embodiment may include a plurality of transducer elements to perform conversion between an electrical signal and an ultrasound signal, transmit the ultrasound signal to an object, and receive an ultrasound echo signal reflected from the object.

Since the ultrasound waves are reflected in a varied degree according to the medium, the transducer module 210 may acquire information about the internal state of the object by collecting the ultrasound echo signals.

That is, the transducer module 210 may generate an ultrasound signal according to a pulse signal or an alternating current applied from a power source, and irradiate the ultrasound signal onto an object. The ultrasound signal irradiated onto the object is reflected from a targeted part inside the object as an ultrasound echo signal. The transducer module 210 receives the reflected ultrasound echo signal, and converts the received ultrasound echo signal into an electrical signal.

To this end, the transducer module 210 includes a piezoelectric layer in which a piezoelectric material vibrates to execute conversion between an electrical signal and an acoustic signal, a matching layer reducing an acoustic impedance difference between the piezoelectric layer and the human body so as to maximally transmit ultrasound waves generated from the piezoelectric layer to a target site of the human body, a lens layer concentrating ultrasound waves proceeding in the forward direction of the piezoelectric layer on a predetermined point, and a backing layer preventing ultrasound waves from proceeding in the backward direction of the piezoelectric layer to prevent image distortion.

In addition, the transducer module 210 is supplied with power from an external power supply device or an internal power storage device, such as a battery. When supplied with power, piezoelectric vibrators or thin films forming the transducer module 210 vibrate. The transducer module 210 irradiates ultrasound waves generated due to the vibration of the piezoelectric vibrators or thin films to an object. Upon receiving ultrasound echo waves reflected from the object, the piezoelectric vibrators or thin films vibrate according to the received ultrasound echo signal. The transducer module 210 generates alternating current at a predetermined frequency corresponding to the vibration frequency of the piezoelectric vibrators or thin films, so that the ultrasound echo signal is converted into an electrical signal.

As such, the transducer module 210 includes a plurality of transducer elements for transmitting the ultrasound signal and receiving the ultrasound echo signal.

The transducer element may be provided, for example, using any one of a magnetostrictive ultrasonic transducer using a magnetostrictive effect of a magnetic body, a piezoelectric ultrasonic transducer or a piezoelectric micromachined ultrasound transducer (pMUT) using a piezoelectric effect of a piezoelectric material, or a capacitive micromachined ultrasonic transducer (cMUT), which transmits and receives ultrasonic waves using vibration of several hundreds or several thousands of micromachined thin films.

The main body 100 according to the embodiment may include a transmission signal generator 110 generating a transmission signal for generating an ultrasound signal irradiated onto an object, a beamformer 120 beamforming a received signal, an inputter 130 receiving an input from a user, a controller 140 determining a numeric value indicating the degree of delivery progression, a display 150 displaying an ultrasound image and the numeric value indicating the degree of delivery progression, and a storage 160 storing various types of information required for controlling the ultrasound imaging apparatus 10.

The transmission signal generator 110 according to the embodiment may generate a transmission signal according to a control command of the controller 140, and transmit the generated transmission signal to the ultrasound probe 200. Here, the transmission signal corresponds to a high voltage electrical signal for vibrating the plurality of transducer elements of the ultrasound probe 200.

The beamformer 120 according to the embodiment may perform conversion between an analog signal and a digital signal to convert a transmission signal (a digital signal) generated from the transmission signal generator 110 into an analog signal or convert an ultrasound echo signal (an analog signal) received from the ultrasound probe 200 into a digital signal, thereby supporting communication between the ultrasound probe 200 and the main body 100.

In addition, the beamformer 120 may add a time delay to the digital signal in consideration of the position of the vibrator and the focus point in order to overcome a difference in times taken for ultrasound waves to reach the focus point or a difference in times taken for ultrasound waves from the focus point to reach the vibrators

That is, assuming that a plurality of transducer elements irradiate ultrasound signals, and a process of collecting all the ultrasound signals at a focus point is referred to as a focusing, the beamformer 120 may perform transmit focusing such that ultrasound signals generated from respective transducer elements are irradiated in a proper order suitable for overcoming the difference in times taken for the ultrasound signals to reach the focus point, and perform receive focusing such that ultrasound echo signals are arranged at the same time by assigning a proper time interval suitable for overcoming the difference in times taken for the ultrasound echo signals to reach the respective transducer elements.

The transmission signal generator 110 and the beamformer 120 may be included in the main body 100 as shown in FIG. 2, but may be provided in the ultrasound probe 200 and perform the function.

The inputter 130 according to the embodiment may receive an input relating to control of the ultrasound imaging apparatus 10 from a user.

In detail, the inputter 130 may receive, for example, a mode selection command regarding an amplitude (A) mode, a brightness (B) mode, a motion (M) mode, or a Doppler image.

In addition, the inputter 130 may receive a user input for adjusting state information of a cervix and position of a fetus. That is, when the state information of the cervix and the position information of the fetus are displayed through the display 150, the user may adjust at least one of the state information of the cervix or the position information of the fetus according to content diagnosed by the user through the inputter 130.

In addition, the inputter 130 may receive a command to start ultrasound image photography from a user, or may receive a photographing protocol (e.g., a photographing part, a photographing time, etc.).

To this end, the inputter 130 may be provided in the main body 100 of the ultrasound imaging apparatus 10, for example, using a physical button, a knob, a touch pad, a touch screen, a stick type manipulation device, a trackball foot switch, a foot pedal, or the like. In this case, the inputter 130 provided as a touch pad or a touch screen may be provided on the display 150.

In addition, the inputter 130 may be provided as a separate input device, such as a keyboard or a mouse, connected to the ultrasound imaging apparatus 10 in a wired or wireless manner.

In addition, the foot switch or foot pedal may be provided at a lower side of the main body 100, and the user may control the operation of the ultrasound imaging apparatus 10 using the foot switch or the foot pedal.

The controller 140 according to the embodiment may generate an ultrasound image on the basis of the ultrasound echo signal received from the ultrasound probe 200.

In detail, the controller 140 may generate an ultrasound image through a scan conversion on the ultrasound echo signal.

Here, the ultrasound image may include not only a gray scale image obtained by scanning an object in A mode, B mode, and M mode, but also a Doppler image representing a moving object using a Doppler effect.

The Doppler image may include a blood flow Doppler image (a color Doppler image) representing a blood flow, a tissue Doppler image representing a tissue movement, and a spectral Doppler image displaying a moving speed of an object as a waveform.

In this case, the user may photograph the transabdominal portion of the object (a mother) or the internal state of the body of the object (a mother) through the ultrasound probe 200 so that a numerical value for the degree of delivery progression may be determined, and the controller 140 may generate an ultrasound image of the cervix of the object (a mother) and the fetus on the basis of ultrasound echo signals received from the ultrasound probe 200. That is, the controller 140 may generate an ultrasound image in which at least one of the cervix or the fetus is displayed on the basis of ultrasound echo signals from the ultrasound probe 200.

According to the embodiment, the controller 140 may process the ultrasound image of the cervix and the fetus to acquire the state information of the cervix and the position information of the fetus.

That is, the controller 140 may identify the cervix and the fetus on the basis of image processing of the ultrasound image based on an image processing algorithm, and acquire state information of the cervix and position information of the fetus on the basis of the identified cervix and fetus.

The image processing algorithm may employ edge detection or morphological operations, and may employ various image processing algorithms without limitation as long as it can identify an object in an ultrasound image.

The state information of the cervix includes the length of the cervix 50, the degree to which the cervix 50 is dilated, the stiffness of the cervix 50, and the position of the cervix 50.

As such, the controller 140 may acquire the state information of the cervix and the position information of the fetus on the basis of echo signals received from at least one of the first probe inserted into the body of an object (a mother) to detect an echo signal or the second probe that comes in contact with the body surface (a transabdominal portion) of an object (a mother) to detect an echo signal.

For example, the controller 140 may acquire the position of the cervix, the stiffness of the cervix, and the length of the cervix by processing the ultrasound image photographed through the first probe, and acquire the degree to which the cervix is dilated and the position of the fetus by processing the ultrasound image photographed through the second probe.

However, the disclosure is not limited to the above example, and the controller 140 may acquire the length of the cervix, the degree to which the cervix is dilated, the stiffness of the cervix, the position of the cervix, and the position of the fetus by processing an ultrasound image photographed from one of the first probe and the second probe.

The controller 140 according to the embodiment may determine a numerical value indicating the degree of delivery progression on the basis of the state information of the cervix and the position information of the fetus.

In this case, the numerical value indicating the degree of delivery progression may correspond to a bishop score, and may be used to predict a delivery time or determine a delivery method by identifying the degree of delivery progression.

In detail, the controller 140 may determine a numerical value indicating the degree of delivery progression by summing numerical values corresponding respectively to the length of the cervix, the degree to which the cervix is dilated, the stiffness of the cervix, the position of the cervix, and the position information of the fetus.

The controller 140 according to an embodiment, in response to the information regarding each of the length of the cervix, the stiffness of the cervix, and the position of the cervix being acquired by processing the ultrasound image, may determine the numerical value corresponding to the degree to which the cervix is dilated to the minimum value.

That is, the controller 140, upon identifying that the cervix has not effaced based on the information about each of the length of the cervix, the stiffness of the cervix, and the position of the cervix being acquired, may identify that the cervix is not dilated, and thus determine that the numerical value corresponding to the degree to which the cervix is dilated to the minimum value.

In addition, the controller 140 according to an embodiment, in response to the information regarding the degree to which the cervix is dilated being acquired by processing the ultrasound image, may determine the numeric values corresponding respectively to the length of the cervix, the stiffness of the cervix, and the position of the cervix to the maximum values.

That is, the controller 140, upon identifying that the cervix is dilated based on the information about the degree to which the cervix is dilated being acquired, may identify that the cervix has effaced and thus determine the numerical values corresponding respectively to the length of the cervix, the stiffness of the cervix, and the position of the cervix to the maximum values.

The controller 140 according to the embodiment may control the display 150 to display a numerical value indicating the degree of delivery progression.

In addition, the controller 140 according to the embodiment may control the display 150 to display at least one of the state information of the cervix or the position information of the fetus.

In this case, the controller 140 according to an embodiment, in response to a user input for adjustment of at least one of the state information of the cervix or the position information of the fetus through the inputter 130 being received, may adjust the at least one of the state information of the cervix or the position information of the fetus on the basis of the user input.

That is, when the state information of the cervix and the position information of the fetus are displayed through the display 150, the user may adjust at least one of the state information of the cervix or the position information of the fetus according to contents diagnosed by the user through the inputter 130.

In this case, the controller 140 may determine the numerical value indicating the degree of delivery progression on the basis of the state information of the cervix and the position information of the fetus adjusted by the user input.

The controller 140 may include at least one memory for storing a program for performing the above-described operations and operations to be described below, and at least one processor for executing the stored program.

The display 150 according to the embodiment may display the ultrasound image generated by the controller 140 and display the numerical value indicating the degree of delivery progression.

In addition, the display 150 according to an embodiment may display at least one of the state information of the cervix or the position information of the fetus.

To this end, the display 150 may be provided on the main body 100 of the ultrasound imaging apparatus 10, and may be provided as a separate display device connected to the ultrasound imaging apparatus 10 in a wired or wireless manner.

The display 150 may include, for example, a liquid crystal display (LCD), a light-emitting diode (LED) display, an organic light-emitting diode (OLED) display, a microelectromechanical system (MEMS) display, or an electronic paper display. However, the type of the display 150 is not limited to the above examples, and the display 150 may be provided as various types of display capable of displaying the numeric value indicating the degree of delivery progression to a user.

The storage 160 according to the embodiment may store various pieces of information required for controlling the ultrasound imaging apparatus 10. For example, the storage 160 may store an ultrasound image generated through the controller 140, an image processing algorithm for processing the ultrasound image, information regarding a correlation between the state information of the cervix and the numeric value of the state information of the cervix, information regarding a correlation between the position information of the fetus and the numeric value of the position information of the fetus, and the like.

To this end, the storage 160 may include a nonvolatile memory device, such as a cache, a read only memory (ROM), a programmable ROM (PROM), an erasable programmable ROM (EPROM), an electrically erasable programmable ROM (EEPROM), and a flash memory, a volatile memory device, such as a random access memory (RAM), or other storage media, such as a hard disk drive (HDD), a CD-ROM, and the like., but the storage 160 is not limited thereto, and may be variously implemented as long as it can store various types of information.

In addition, the storage 160 may be a memory implemented as a chip separated from the processor, which has been described above in connection with the controller 140, or may be implemented as a single chip integrated with the processor.

Meanwhile, the ultrasound imaging apparatus 10 may further include a communicator (not shown). In this case, the communicator may be connected to a network in a wired or wireless manner to communicate with an external device or a server.

The communicator may exchange data with a hospital server or another medical device in a hospital connected through a PACS, and perform data communication according to digital imaging and communications in medicine (DICOM) standard.

In addition, the communicator may perform data communication with a portable terminal of a doctor or a patient, as well as a server or a medical device in a hospital.

The communicator may transmit and receive data related to diagnosis of an object, such as an ultrasound image, echo ultrasound data, and Doppler data of the object through a network, and may also transmit and receive medical images taken by other medical devices, such as computed tomography (CT), magnetic resonance imaging (MRI), and X-ray imaging.

In addition, the communicator may receive information regarding a diagnosis history, a treatment schedule, and the like of a patient from the server, and use the information to diagnose the object.

To this end, the communicator may be connected to a network in a wired or wireless manner to exchange data with a server, a medical device, or a portable terminal. The communicator may include one or more components that enable communication with an external device, and may include, for example, a short-range communication module, a wired communication module, and a mobile communication module.

In the above, each configuration of the ultrasound imaging apparatus 10 has been described. Hereinafter, determination of the numerical value indicating the degree of delivery progression by the ultrasound imaging apparatus 10 will be described in detail.

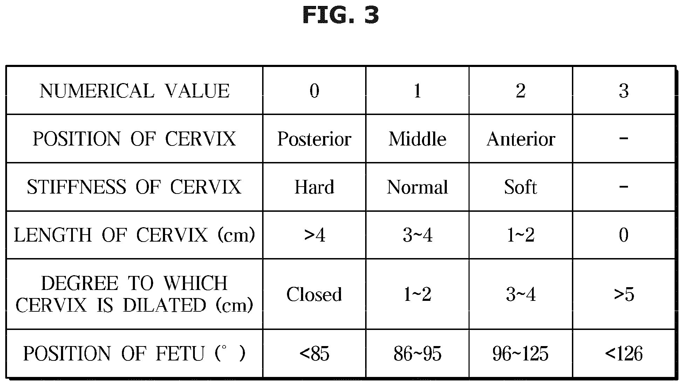

FIG. 3 is a diagram for describing a case in which the ultrasound imaging apparatus 10 according to the embodiment determines the numerical value indicating the degree of delivery progression.

Referring to FIG. 3, the controller 140 according to the embodiment may generate an ultrasound image on the basis of ultrasound echo signals received from the ultrasound probe 200.

In detail, the controller 140 may generate the ultrasound image through a scan conversion process for ultrasound echo signals.

In this case, the user may photograph a transabdominal portion of an object (a mother) or an internal state of the body of an object (a mother) through the ultrasound probe 200 so that the numerical value for the degree of delivery progression may be determined, and the controller 140 may generate an ultrasound image of the cervix of the object (a mother) and the fetus on the basis of ultrasound echo signals received from the ultrasound probe 200. That is, the controller 140 may generate an ultrasound image in which at least one of the cervix or the fetus is displayed on the basis of ultrasound echo signals from the ultrasound probe 200.

The controller 140 according to the embodiment may process the ultrasound image of the cervix and the fetus to acquire state information of the cervix and position information of the fetus.

That is, the controller 140 may identify the cervix and the fetus on the basis of image processing of the ultrasound image based on an image processing algorithm, and acquire state information of the cervix and position information of the fetus on the basis of the identified cervix and fetus.

The image processing algorithm may employ edge detection or morphological operations, and employ various image processing algorithms without limitation as long as it can identify an object in an ultrasound image.

The controller 140 according to the embodiment may determine a numerical value indicating the degree of delivery progression on the basis of the state information of the cervix and the position information of the fetus.

In this case, the numerical value indicating the degree of delivery progression may correspond to a bishop score, and may be used to predict a delivery time or determine a delivery method by identifying the degree of delivery progression.

In detail, the controller 140 may determine the numerical value indicating the degree of delivery progression by summating numerical values corresponding respectively to the length of the cervix, the degree to which the cervix is dilated, the stiffness of the cervix, the position of the cervix, and the position information of the fetus.

In this case, the storage 160 may store information about a correlation between the state information of the cervix and the numerical value of the state information of the cervix, and information about a correlation between the position information of the fetus and the numerical value of the position information of the fetus, and the controller 140 may determine the numerical values corresponding respectively to the length of the cervix, the degree to which the cervix is dilated, the stiffness of the cervix, the position of the cervix, and the position information of the fetus on the basis of the information about the correlation.

The position of the cervix may be shifted forward toward the vagina of the object as the fetus descends toward the pelvis. Accordingly, the numerical value corresponding to the position of the cervix may be determined to be higher as the position of the cervix is shifted forward toward the vagina of the object, as shown in FIG. 3.

The stiffness of the cervix may weaken as delivery progresses. Accordingly, the numerical value corresponding to the stiffness of the cervix may be determined to be higher as the stiffness of the cervix becomes weaker, as shown in FIG. 3.

The length of the cervix may shorten as delivery progresses. Accordingly, the numerical value corresponding to the length of the cervix may be determined to be higher value as the length of the cervix decreases, as shown in FIG. 3. For example, the cervix having a length of 4cm or more is assigned with a numerical value "0", and the cervix having a length of 3cm to 4cm is assigned with a numeric value "1", and the cervix having a length of 1cm to 2cm is assigned with a numerical value "2", and the cervix having a length completely effaced to 0cm is assigned with a numerical value "3".

The degree to which the cervix is dilated may increase as delivery progresses. Accordingly, the numerical value corresponding to the degree to which the cervix is dilated may be determined to higher as the degree to which the cervix is dilated increases, as shown in FIG. 3. For example, the cervix when closed is assigned with a numerical value "0", and the cervix dilated from 1cm to 2cm is assigned with a numerical value "1", and the cervix dilated from 3cm to 4cm is assigned with a numerical value "2", and the cervix dilated by 5cm or more is assigned with a numerical value "3".

The position of the fetus may be shifted downward toward the pelvis as the delivery progresses. In this case, the position of the fetus may be defined on the basis of the angle of progress (AOP) between the long axis of the pubic bone and the line contacting from the lowermost edge of the pubic bone to the fetal skull, and a larger AOP represents more progression of delivery. Accordingly, the numerical value corresponding to the position information of the fetus may be determined to be higher as the AOP increases, as shown in FIG. 3. For example, an AOP of 85° or less is assigned with a numerical value "0", an AOP from 86° to 95° is assigned with a numeric value "1", and an AOP from 96° to 125° is assigned with a numeric value "2", and an AOP of 126° or more is assigned with a numeric value "3".

According to an embodiment, the controller 140, in response to the information regarding each of the length of the cervix, the stiffness of the cervix, and the position of the cervix being acquired by processing the ultrasound image, may determine the numerical value corresponding to the degree to which the cervix is dilated to the minimum value.

That is, the controller 140, upon identifying that the cervix has not effaced based on the information about each of the length of the cervix, the stiffness of the cervix, and the position of the cervix being acquired, may identify that the cervix is not dilated, and thus determine that the numerical value corresponding to the degree to which the cervix is dilated to the minimum value.

In addition, according to an embodiment, the controller 140, in response to the information regarding the degree to which the cervix is dilated being acquired by processing the ultrasound image, may determine the numeric values corresponding respectively to the length of the cervix, the stiffness of the cervix, and the position of the cervix to the maximum values.

That is, the controller 140, upon identifying that the cervix is dilated based on the information about the degree of cervix dilatation being acquired, may identify that the cervix has effaced and thus determine the numerical values corresponding respectively to the length of the cervix, the stiffness of the cervix, and the position of the cervix to the maximum values.

In the above, the determination of the numerical value indicating the degree of delivery progression on the basis of the state information of the cervix and the position information of the fetus has been described in detail. Hereinafter, acquisition of the state information of the cervix and the position information of the fetus will be described in detail.

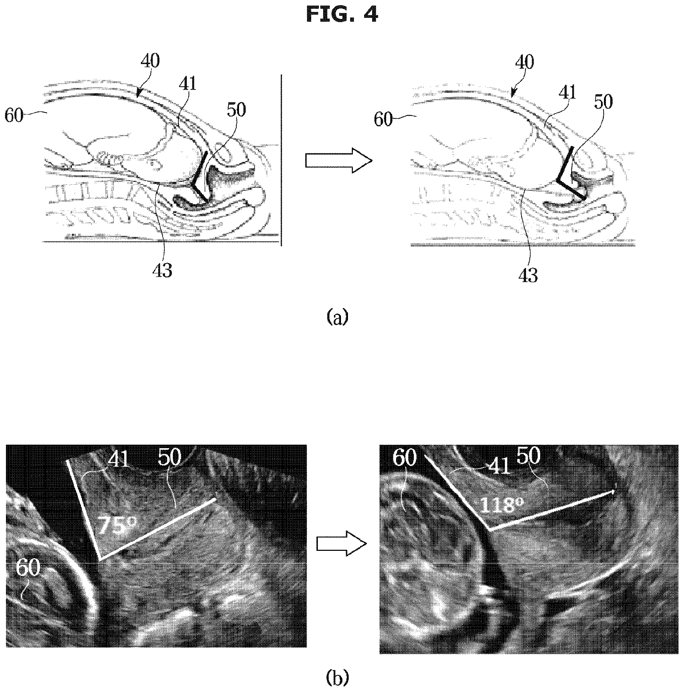

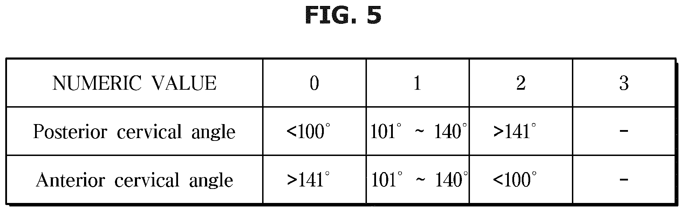

FIGS. 4A and 4B are diagrams illustrating a case in which the ultrasound imaging apparatus 10 according to the embodiment determines the position of a cervix, and FIG. 5 is a diagram illustrating numerical values for the positions of the cervix.

Referring to FIG. 4, the position of a cervix 50 may be shifted forward toward the vagina of an object as a fetus 60 descends toward the pelvis (see FIG. 4A).

In this case, the angle between an anterior wall 41 of a uterus 40 and the cervix 50 may decrease as the position of the cervix 50 is shifted forward. In addition, the angle between a posterior wall 43 of the uterus 40 and the cervix 50 may increase as the position of the cervix 50 is shifted forward.

For example, referring to FIG. 4, the controller 140 according to the embodiment may determine the angle between the anterior wall 41 of the uterus 40 and the cervix 50 as the position of the cervix 50 on the basis of processing of the ultrasound image, and may determine a numerical value corresponding to the angle as the numerical value corresponding to the position of the cervix 50 (see FIG. 4B).

That is, the controller 140 may determine the position of the cervix on the basis of the angle between the anterior wall 41 of the uterus 40 and the cervix 50 or the angle between the posterior wall 43 of the uterus 40 and the cervix 50 from the ultrasound image, and determine the numerical value corresponding to the position of the cervix.

For example, referring to FIG. 5, when the numeric value corresponding to the position of the cervix 50 is determined on the basis of the angle between the anterior wall 41 of the uterus 40 and the cervix 50, the controller 140 may determine the numerical value corresponding to the position of the cervix 50 to be higher as the angle decreases on the basis of boundaries of 100° and 140°. That is, the controller 140 may assign an angle less than 100° with a numeric value "0", an angle of 101° to 140° with a numeric value "1", and an angle of 141° or more with a numeric value "2".

In addition, for example, referring to FIG. 5, when the numeric value corresponding to the position of the cervix is determined on the basis of the angle (the posterior cervical angle) between the posterior wall 43 of the uterus 40 and the cervix 50, the controller 140 may determine the numerical value corresponding to the position of the cervix to be higher as the angle increase on the basis of boundaries of 100° and 140°. That is, the controller 140 may determine an angle less than 100° with a numeric value "2", an angle of 101° to 140° with a numeric value "1"h, and an angle of 141° or more with a numeric value "0".

In this case, the cervix 50 may be effaced as the delivery progresses, and when the angle with respect to the anterior wall 41 or the posterior wall 43 of the uterus 40 is not determinable due to the effacement of the cervix 50, the controller 140 may determine the numerical value corresponding to the position of the cervix 50 as the maximum value (e.g., "2").

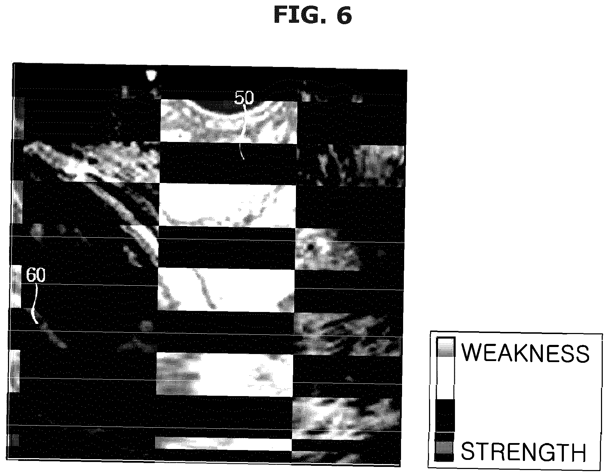

FIG. 6 is a diagram illustrating a case in which the ultrasound imaging apparatus 10 according to the embodiment of the disclosure determines stiffness of the cervix.

Referring to FIG. 6, the controller 140 according to the embodiment may measure a stiffness at a photographing site on the basis of echo signals from the ultrasound probe 200. That is, the controller 140 may measure the stiffness at the photographing site using material properties in which reflected echo signals dare varied with the stiffness of the material, and may display the stiffness in the ultrasound image in different colors or contrasts according to the stiffness.

The stiffness of the cervix 50 may weaken as delivery progresses. Accordingly, the controller 140 may determine the numerical value to be higher as the stiffness of the cervix 50 becomes weaker. In detail, assuming a first set value and a second set value as boundaries, the controller 140 may assign a stiffness greater than or equal to the first set value (Hard) with a numeric value "0", a stiffness between the first set value and the second set value (Normal) with a numeric value "1", and a stiffness less than the second set value (Soft) with a numeric value "2".

In this case, the cervix 50 may be effaced as the delivery progresses, and when the stiffness of the cervix 50 is not determinable due to effacement of the cervix 50, the controller 140 may determine the numerical value corresponding to the stiffness of the cervix 50 as the maximum value (e.g., "2").

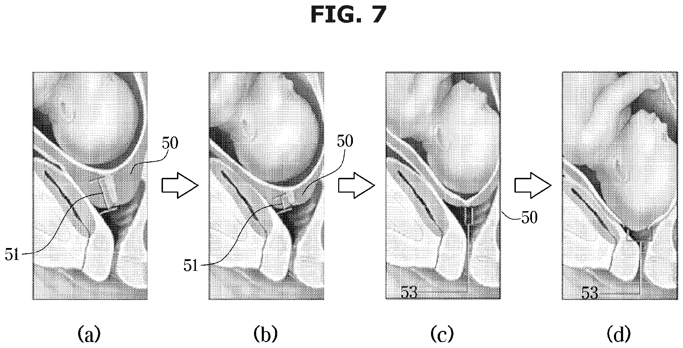

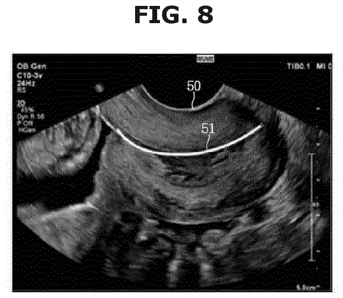

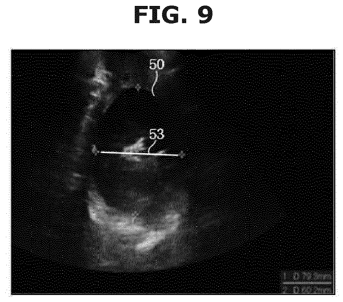

FIG. 7 is a diagram for describing the length of the cervix 50 and the degree to which the cervix 50 is dilated, FIG. 8 is a diagram illustrating a case in which the ultrasound imaging apparatus 10 according to the embodiment of the disclosure determines the length of the cervix 50, and FIG. 9 is a diagram illustrating a case in which the ultrasound imaging apparatus 10 according to the embodiment determines the degree to which the cervix 50 is dilated.

Referring to FIGS. 7A to 7D, a length 51 of the cervix 50 may be shortened due to the effacement of the cervix 50 as delivery proceeds. In addition, the degree 53 to which the cervix 50 is dilated may increase due to the effacement of the cervix 50 as delivery progresses.

In this case, FIGS. 7A to 7D sequentially show changes in the length 51 of the cervix 50 and the degree 53 to which the cervix 50 is dilated as delivery progresses.

As such, the length 51 of the cervix 50 may become shorter in the order of FIG. 7A, FIG. 7B, FIG. 7C, and FIG. 7D, and in FIG. 7D, a numeric value "0" may be assigned due to the effacement of the cervix 50.

In addition, the degree 53 to which the cervix 50 is dilated may increase in the order of FIG. 7A, FIG. B, FIG. C, and FIG. 7D, for example, when the cervix 50 is dilated by 3 cm in FIG. 7C, the cervix 50 may dilated by 10 cm in FIG. 7D.

Referring to FIG. 8, the controller 140 according to the embodiment may determine the length 51 of the cervix 50 by processing the ultrasound image, and determine the numeric value corresponding to the length 51 of the cervix 50 by comparing the length of the cervix 51 with boundary reference values (e.g., 1 cm, 2 cm, 3 cm, and 4cm).

Referring to FIG. 9, the controller 140 according to the embodiment may determine the degree 53 to which the cervix 50 is dilated by processing the ultrasound image, and in this case, the degree 53 to which the cervix 50 is dilated may correspond to the degree to which the cervix 50 is open, and may be expressed as a diameter in the open area. The controller 140 determines the numerical value corresponding to the degree 53 to which the cervix 50 is dilated by comparing the diameter in the open area with the boundary reference values (e.g., 1 cm, 2 cm, 3 cm, 4 cm, and 5cm).

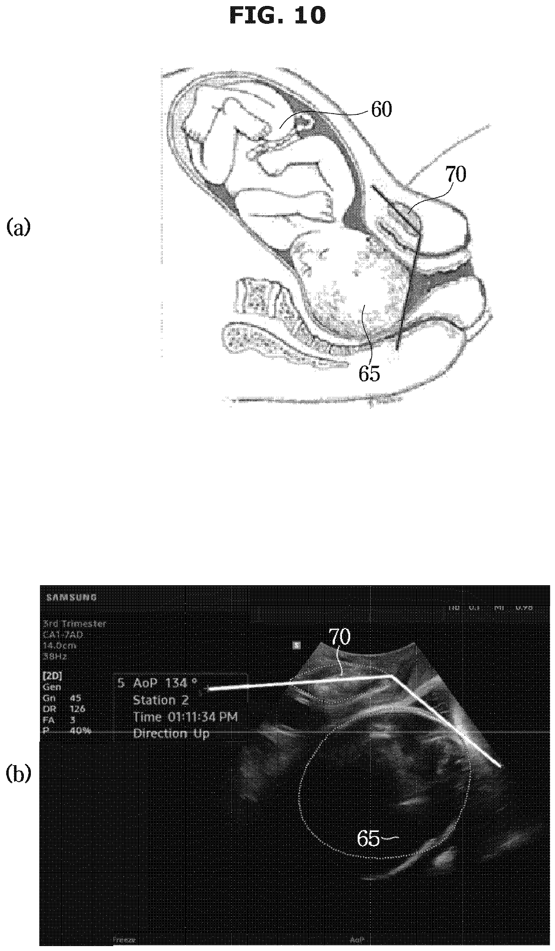

FIGS. 10A and 10B are diagrams illustrating a case in which the ultrasound imaging apparatus 10 according to the embodiment of the disclosure determines position information of a fetus.

Referring to FIGS. 10A and 10B, the position of a fetus 60 may be shifted downward toward the pelvis as the delivery progresses. In this case, the position of the fetus 60 may be defined on the basis of the angle of progress (AOP) between the long axis of the pubic bone 70 and the line contacting from the lowermost edge of the pubic bone 70 to the fetal skull, and a larger AOP represents more progression of delivery (see FIG. 10A)

The controller 140 according to the embodiment may calculate the AOP by processing the ultrasound image, and may determine the numerical value corresponding to the position information of the fetus 60 by comparing the AOP with boundary reference values (e.g., 85°, 95°, and 125°).

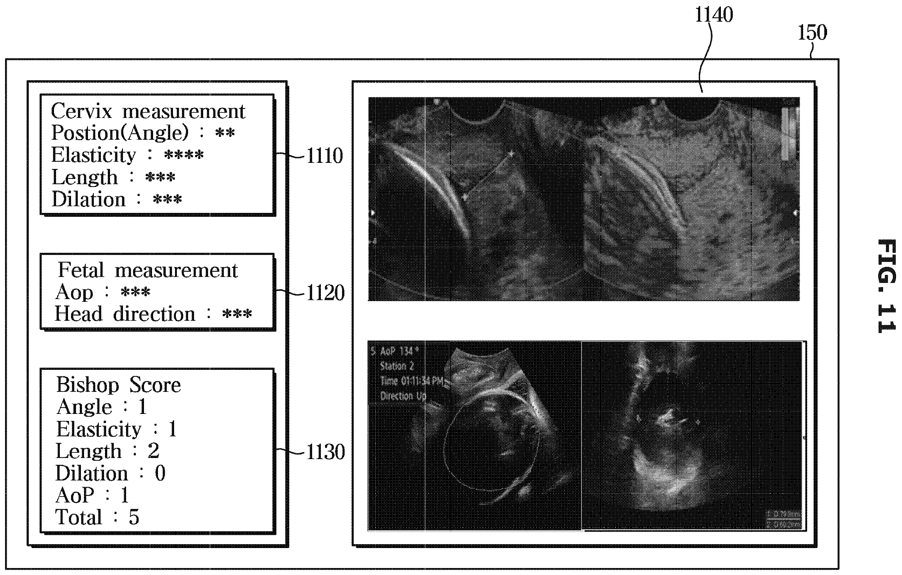

FIG. 11 is a diagram illustrating a case in which the ultrasound imaging apparatus 10 according to the embodiment of the disclosure displays a numerical value indicating the degree of delivery progression.

Referring to FIG. 11, the controller 140 according to the embodiment may control the display 150 to display the numerical value indicating the degree of delivery progression.

With such a configuration, the display 150 may display information 1130 about the numerical value. In detail, the display 150 may display the numerical value (Total) indicating the degree of delivery progression.

In this case, according to an embodiment, the information 1130 about the numerical value may include information about details of the numerical value (Total) indicating the degree of delivery progression. That is, the controller 140 may control the display 150 to display numerical values corresponding respective to the state information of the cervix 50 and the position information of the fetus 60.

For example, the display 150 may display, as the information about details of the numerical value (Total) indicating the degree of delivery progression, a numerical value (Angle: 1) corresponding to the position of the cervix 50, a numerical value (Elasticity: 1) corresponding to the stiffness of the cervix 50, a numerical value (Length: 1) corresponding to the length of the cervix 50, a numerical value (Dilation: 0) corresponding to the degree to which the cervix 50 is dilated, and a numerical value (AOP: 1) corresponding to the position information of the fetus 60.

In addition, the controller 140 according to the embodiment may control the display 150 to display at least one of the state information of the cervix 50 or the position information of the fetus 60.

With such a configuration, the display 150 may display at least one of state information 1110 of the cervix 50 or position information 1120 of the fetus 60.

For example, the display 150 may display the position (Position (Angle)) of the cervix 50, the stiffness (Elasticity) of the cervix 50, the length (Length) of the cervix 50, and the degree of dilation (Dilation) of the cervix 50 as the state information 1110 of the cervix 50.

In addition, the display 150 may display AOP as the position information 1120 of the fetus 60, and may further display a head direction (Head direction) according to an embodiment.

The controller 140 according to the embodiment may control the display 150 to display an ultrasound image 1140.

Accordingly, the display 150 may display the ultrasound image 1140 according to embodiments. In this case, the ultrasound image 1140 may include an ultrasound image processed to acquire at least one of state information of the cervix 50 or position information of the fetus 60, and may include a plurality of ultrasound images including at least one of the cervix 50 or the fetus 60.

The controller 140 according to an embodiment, in response to a user input for adjusting at least one of the state information of the cervix 50 or the position information of the fetus 60 being received through the inputter 130, may adjust at least one of the state information of the cervix 50 or the position information of the fetus 60 on the basis of the user input.

In other words, when the state information of the cervix 50 and the position information of the fetus 60 are displayed through the display 150, the user may adjust at least one of the state information of the cervix 50 or the position information of the fetus 60 according to contents diagnosed by the user through the inputter 130. In this case, the user's diagnosis may refer to diagnosis obtained through endoscopy or user's analysis of the ultrasound image 1140 displayed on the display 150 according to an embodiment.

In this case, the controller 140 may determine the numerical value indicating the degree of delivery progression on the basis of the state information of the cervix 50 and the position information of the fetus 60 adjusted by the user input.

Hereinafter, a method of controlling the ultrasound imaging apparatus 10 according to an embodiment will be described. The method of controlling the ultrasound imaging apparatus 10 to be described below may employ the ultrasound imaging apparatus 10 according to the above-described embodiment. Therefore, the description made above with reference to FIGS. 1 to 11 may be equally applied to the method of controlling the ultrasound imaging apparatus 10 according to the embodiment unless otherwise mentioned.

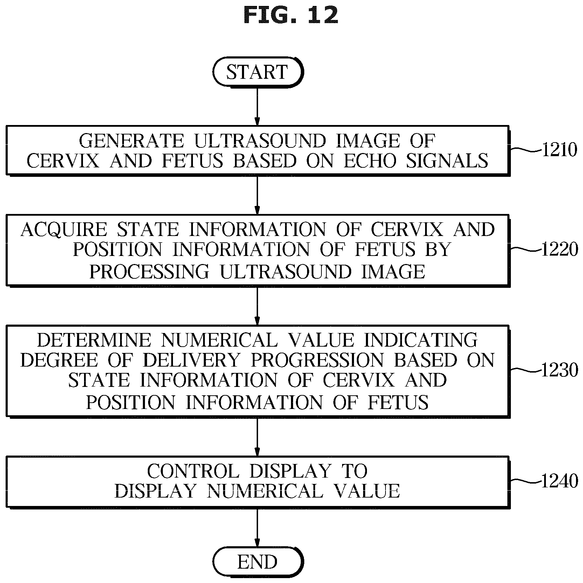

FIG. 12 is a flowchart showing the method of controlling the ultrasound imaging apparatus 10 according to the embodiment of the disclosure, which shows a case of displaying a numerical value indicating the degree of delivery.

Referring to FIG. 12, the ultrasound imaging apparatus 10 according to the embodiment may generate an ultrasound image of the cervix 50 and the fetus 60 on the basis of echo signals (1210).

In this case, a user may photograph the transabdominal portion of the object (a mother) or the internal state of the body of the object (a mother) through the ultrasound probe 200 so that a numerical value for the degree of delivery progression may be determined, and the controller 140 may generate an ultrasound image of the cervix of the object (mother) and the fetus on the basis of ultrasound echo signals received from the ultrasound probe 200. That is, the controller 140 may generate an ultrasound image in which at least one of the cervix or the fetus is displayed on the basis of ultrasound echo signals from the ultrasound probe 200.

The ultrasound imaging apparatus 10 according to the embodiment may process the ultrasound image to acquire state information of the cervix 50 and position information of the fetus 60 (1220).

That is, the controller 140 may identify the cervix 50 and the fetus 60 on the basis of image processing of the ultrasound image based on an image processing algorithm, and acquire state information of the cervix 50 and position information of the fetus 60 on the basis of the identified cervix 50 and fetus 60.

The image processing algorithm may employ edge detection or morphological operations, and may employ various image processing algorithms without limitation as long as it can identify an object in an ultrasound image.

The state information of the cervix 50 includes the length of the cervix 50, the degree to which the cervix 50 is dilated, the stiffness of the cervix 50, and the position of the cervix 50.

The ultrasound imaging apparatus 10 according to the embodiment may determine a numerical value indicating the degree of delivery progression on the basis of the state information of the cervix 50 and the position information of the fetus 60 (1230).

In this case, the numerical value indicating the degree of delivery progression may correspond to a bishop score, and may be used to predict a delivery time or a delivery method by identifying the degree of delivery progression.

In detail, the controller 140 may determine a numerical value indicating the degree of delivery progression by summating numerical values corresponding respectively to the length of the cervix 50, the degree to which the cervix 50 is dilated, the stiffness of the cervix 50, the position of the cervix 50, and the position information of the fetus 60.

The controller 140 according to an embodiment, in response to information regarding each of the length of the cervix 50, the stiffness of the cervix 50, and the position of the cervix 50 being acquired by processing the ultrasound image, may determine the numerical value corresponding to the degree to which the cervix is dilated to the minimum value.

That is, the controller 140, upon identifying that the cervix 50 has not effaced based on the information about each of the length of the cervix 50, the stiffness of the cervix 50, and the position of the cervix 50 being acquired, may identify that the cervix 50 is not dilated, and thus determine that the numerical value corresponding to the degree to which the cervix 50 is dilated to the minimum value.

In addition, the controller 140 according to an embodiment, in response to information regarding the degree to which the cervix 50 is dilated being acquired by processing the ultrasound image, may determine the numeric values corresponding respectively to the length of the cervix 50, the stiffness of the cervix 50, and the position of the cervix 50 to the maximum values.

That is, the controller 140, upon identifying that the cervix 50 is dilated based on information about the degree to which the cervix 50 is dilated being acquired, may identify that the cervix 50 has effaced and thus determine the numerical values corresponding respectively to the length of the cervix 50, the stiffness of the cervix 50, and the position of the cervix 50 to the maximum values.

The ultrasound imaging apparatus 10 according to the embodiment may control the display 150 to display the numerical value indicating the degree of delivery progress (1240).

As such, the ultrasound imaging apparatus 10 may determine and display a Bishop score using ultrasound waves, providing a highly reliable Bishop score to a user

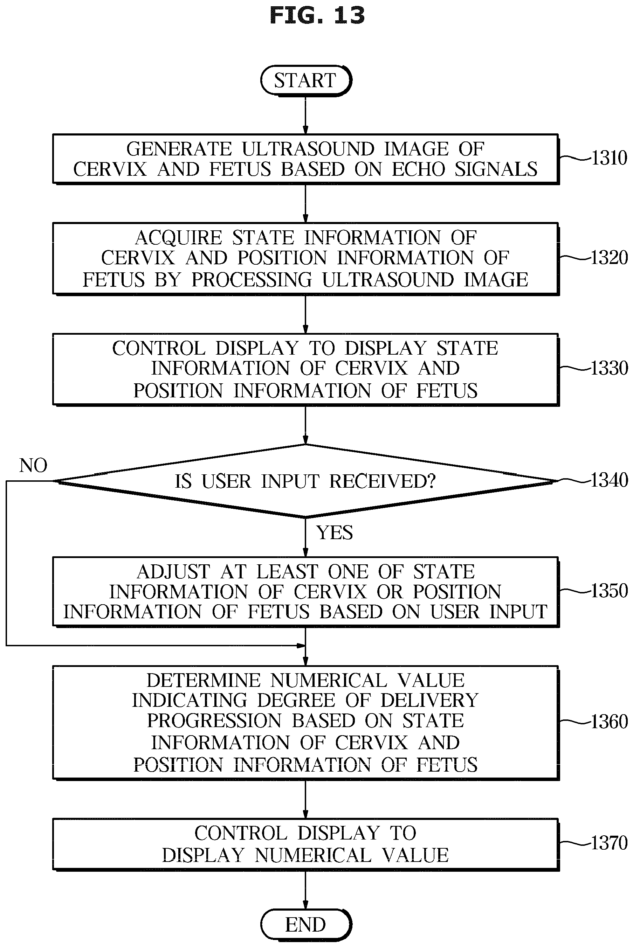

FIG. 13 is a flowchart showing the method of controlling the ultrasound imaging apparatus 10 according to the embodiment of the disclosure, which shows a case of determining a numerical value indicating the degree of delivery progression on the basis of a user input.

Referring to FIG. 13, the ultrasound imaging apparatus 10 according to the embodiment may generate an ultrasound image of the cervix 50 and the fetus 60 on the basis of echo signals (1310), and may acquire state information of the cervix 50 and position information of the fetus 60 by processing the ultrasound image (1320).

In this case, the ultrasound imaging apparatus 10 according to the embodiment may display the state information of the cervix 50 and the position information of the fetus 60 (1330).

The ultrasound imaging apparatus 10 according to an embodiment, in response to a user input for adjustment of at least one of the state information of the cervix 50 or the position information of the fetus 60 being received through the inputter 130 (Yes in operation 1340), may adjust the at least one of the state information of the cervix 50 or the position information of the fetus 60 on the basis of the user input (1350).

That is, when the state information of the cervix 50 and the position information of the fetus 60 are displayed through the display 150, the user may adjust at least one of the state information of the cervix 50 or the position information of the fetus 60 according to contents diagnosed by the user through the inputter 130. In this case, the user's diagnosis according to an embodiment may refer to diagnosis obtained through endoscopy or user' analysis of the ultrasound image 1140 displayed on the display 150.

The ultrasound imaging apparatus 10 according to the embodiment may determine the numerical value indicating the degree of delivery progression on the basis of the state information of the cervix 50 and the position information of the fetus 60 adjusted by the user input (1360).

That is, the controller 140, in response to a user input being received, may determine the numerical value indicating the degree of delivery progression on the basis of the state information of the cervix 50 and the position information of the fetus 60 adjusted by the user input, and in response to a user input not being received, may determine the numerical value indicating the degree of delivery progression on the basis the acquired state information of the cervix 50 and the acquired position information of the fetus 60.

The ultrasound imaging apparatus 10 according to the embodiment may control the display 150 to display the numerical value (1370).

As such, the ultrasound imaging apparatus 10 may determine and display a Bishop score through ultrasound to provide a user with a highly reliable Bishop score, and may provide a user with an interface for adjusting the acquired state information of the cervix 50 and the acquired position information of the fetus 60, to thereby provide an opportunity to obtain a more reliable Bishop score.

Meanwhile, the disclosed embodiments may be embodied in the form of a recording medium storing instructions executable by a computer. The instructions may be stored in the form of program code and, when executed by a processor, may generate a program module to perform the operations of the disclosed embodiments. The recording medium may be embodied as a computer-readable recording medium.

The computer-readable recording medium includes all kinds of recording media in which instructions which may be decoded by a computer are stored, for example, a Read Only Memory (ROM), a Random-Access Memory (RAM), a magnetic tape, a magnetic disk, a flash memory, an optical data storage device, and the like.

As is apparent from the above, the ultrasound imaging apparatus and the method of controlling the same can provide a Bishop score with a high reliability by determining the Bishop score through ultrasound and displaying the Bishop score.

Although embodiments of the disclosure have been described for illustrative purposes, those skilled in the art will appreciate that various modifications, additions and substitutions are possible, without departing from the scope and spirit of the disclosure. Therefore, embodiments of the disclosure have not been described for limiting purposes.

## Claims

1. An ultrasound imaging apparatus comprising:
a display;
a probe configured to transmit an ultrasound signal to an object and detect an echo signal; and
a controller configured to generate an ultrasound image of a cervix of the object and a fetus on the basis of the echo signal, acquire state information of the cervix and position information of the fetus by processing the ultrasound image, determine a numeric value indicating a degree of delivery progression on the basis of the state information of the cervix and the position information of the fetus, and control the display to display the numeral value.

2. The ultrasound imaging apparatus of claim 1, wherein the state information of the cervix includes a length of the cervix, a degree to which the cervix is dilated, a stiffness of the cervix, and a position of the cervix.

3. The ultrasound imaging apparatus of claim 2, wherein the controller determines the numeral value indicating the degree of delivery progression by summating numeral values corresponding respectively to the length of the cervix, the degree to which the cervix is dilated, the stiffness of the cervix, the position of the cervix, and the position information of the fetus.

4. The ultrasound imaging apparatus of claim 3, wherein the controller, in response to information regarding each of the length of the cervix, the stiffness of the cervix, and the position of the cervix being acquired by processing the ultrasound image, determines the numeric value corresponding to the degree to which the cervix is dilated to a minimum value.

5. The ultrasound imaging apparatus of claim 3, wherein the controller, in response to the degree to which the cervix is dilated being acquired by processing the ultrasound image, determines the numeric values corresponding respectively to the length of the cervix, the stiffness of the cervix, and the position of the cervix to maximum values.

6. The ultrasound imaging apparatus of claim 2, wherein the probe includes: a first probe configured to be inserted into a body of the object to detect the echo signal; and
a second probe configured to come in contact with a surface of the object to detect the echo signal.

7. The ultrasound imaging apparatus of claim 6, wherein the controller acquires the position of the cervix, the stiffness of the cervix, and the length of the cervix by processing the ultrasound image generated on the basis of the echo signal of the first probe.

8. The ultrasound imaging apparatus of claim 6, wherein the controller acquires the degree to which the cervix is dilated and the position information of the fetus by processing the ultrasound image generated on the basis of the echo signal of the second probe.

9. The ultrasound imaging apparatus of claim 1, wherein the controller controls the display to display at least one of the state information of the cervix or the position information of the fetus.

10. The ultrasound imaging apparatus of claim 1, further comprising an inputter configured to receive a user input,
wherein the controller adjusts at least one of the state information of the cervix or the position information of the fetus on the basis of the user input received through the inputter.

11. A method of controlling an ultrasound imaging apparatus including a display and a probe configured to transmit an ultrasound signal to an object and detect an echo signal, the method comprising:
generating an ultrasound image of a cervix of the object and a fetus on the basis of the echo signal;
acquiring state information of the cervix and position information of the fetus by processing the ultrasound image;
determining a numeric value indicating a degree of delivery progression on the basis of the state information of the cervix and the position information of the fetus; and
controlling the display to display the numeral value.

12. The method of claim 11, wherein the state information of the cervix includes a length of the cervix, a degree to which the cervix is dilated, a stiffness of the cervix, and a position of the cervix.

13. The method of claim 12, wherein the determining of the numeric value indicating the degree of delivery progression on the basis of the state information of the cervix and the position information of the fetus includes determining the numeral value indicating the degree of delivery progression by summating numeral values corresponding respectively to the length of the cervix, the degree to which the cervix is dilated, the stiffness of the cervix, the position of the cervix, and the position information of the fetus.

14. The method of claim 13, wherein the determining of the numeric value indicating the degree of delivery progression on the basis of the state information of the cervix and the position information of the fetus includes, in response to information regarding each of the length of the cervix, the stiffness of the cervix, and the position of the cervix being acquired by processing the ultrasound image, determining the numeric value corresponding to the degree to which the cervix is dilated to a minimum value.

15. The method of claim 13, wherein the determining of the numeric value indicating the degree of delivery progression on the basis of the state information of the cervix and the position information of the fetus includes, in response to the degree to which the cervix is dilated being acquired by processing the ultrasound image, determining the numeric values corresponding respectively to the length of the cervix, the stiffness of the cervix, and the position of the cervix to maximum values.
